(19) Europäisches Patentamt
European Patent Office
Office européen des brevets

(11) EP 1 038 526 A1

# (12) EUROPÄISCHE PATENTANMELDUNG

(43) Veröffentlichungstag:
**27.09.2000 Patentblatt 2000/39**

(51) Int. Cl.[7]: **A61K 31/506**, A61K 31/505, A61P 1/00

(21) Anmeldenummer: **00104990.7**

(22) Anmeldetag: **09.03.2000**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **15.03.1999 DE 19911371**

(71) Anmelder:
**Solvay Pharmaceuticals GmbH**
**30173 Hannover (DE)**

(72) Erfinder:
- **Eeckhout, Christian, Dr.**
  **29690 Lindwedel (DE)**
- **Sann, Holger, Dr.**
  **30629 Hannover (DE)**

(74) Vertreter:
**Gosmann, Martin, Dr. et al**
**Solvay Pharmaceuticals GmbH,**
**Patentabteilung,**
**Hans-Böckler-Allee 20**
**30173 Hannover (DE)**

## (54) Behandlung von funktionellen Störungen der unteren Darmwege und einhergehenden visceralen Schmerzen mit Moxonidin

(57) Beschrieben wird die Verwendung von Moxonidin und dessen physiologisch verträglichen Säureadditionssalzen zur Behandlung und/oder Prophylaxe von funktionellen gastrointestinalen Störungen bzw. Erkrankungen im Bereich der unteren Darmwege, insbesondere zur Behandlung und/oder Prophylaxe von damit einhergehenden abdominalen visceralen Schmerzen, und die Herstellung von für diese Behandlung bzw. Prophylaxe geeigneten Arzneimitteln. Die erfindungsgemäß behandelbaren funktionellen Störungen bzw. Erkrankungen der unteren Darmwege sind hierbei insbesondere als IBS und IBD bekannt, bei denen als Krankheitssymptome im allgemeinen auch viscerale Schmerzen auftreten.

EP 1 038 526 A1

Printed by Xerox (UK) Business Services
2.16.7 (HRS)/3.6

## Beschreibung

Die vorliegende Erfindung betrifft die Verwendung von 4-Chlor-5-[(4,5-dihydro-1H-imidazol-2-yl)-amino]-6-methoxy-2-methylpyrimidin (= Moxonidin) und dessen physiologisch verträglichen Säureadditionssalzen zur Behandlung und/oder Prophylaxe von funktionellen gastrointestinalen Störungen und Erkrankungen im Bereich der unteren Darmwege, insbesondere zur Behandlung und/oder Prophylaxe von damit einhergehenden abdominalen visceralen Schmerzen, und zur Herstellung von für diese Behandlung und/oder Prophylaxe geeigneten Arzneimitteln.

Der Erfindung liegt die Aufgabe zugrunde, neue pharmazeutische Zubereitungen zur Behandlung von funktionellen und/oder erkrankungsbedingten Symptomen im Bereich der unteren Darmwege, und insbesondere z.B. zur Behandlung und/oder Prophylaxe von damit einhergehenden abdominalen visceralen Schmerzen, zu entwickeln.

Erfindungsgemäß werden zur Herstellung von pharmazeutischen Zubereitungen zur Behandlung und/oder Prophylaxe von mit erhöhter Schmerzempfindlichkeit im Colonbereich verbundenen funktionellen Störungen und Erkrankungen der unteren Darmwege, vorzugsweise zur Behandlung und/oder Prophylaxe von damit einhergehenden abdominalen visceralen Schmerzen, in größeren Säugetieren und Menschen 4-Chlor-5-[(4,5-dihydro-1H-imidazol-2-yl)-amino]-6-methoxy-2-methylpyrimidin der allgemeinen Formel I,

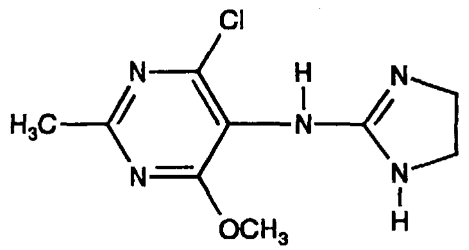

I

und dessen physiologisch verträgliche Säureadditionssalze verwendet.

Als physiologisch verträgliche Säureadditionssalze des Moxonidins eignen sich Salze mit anorganischen Säuren, beispielsweise Halogenwasserstoffsäuren, oder mit organischen Säuren, beispielsweise niederen aliphatischen Mono- oder Dicarbonsäuren wie Essigsäure, Fumarsäure oder Weinsäure oder aromatischen Carbonsäuren wie z.B. Salicylsäure.

Die erfindungsgemäß eingesetzten Verbindungen fallen unter den Umfang von in der deutschen Patentanmeldung Nr. 28 49 537 beschriebenen 5-[(2-Imidazolin-2-yl)-amino]-pyrimidin-Derivaten mit blutdrucksenkenden Eigenschaften, und sind aus dieser Patentanmeldung bekannt. Moxonidinhaltige pharmazeutische Zubereitungen sind als Antihypertensiva unter den Markennamen Physiotens$^R$ im Handel erhältlich und werden medizinisch als Antihypertensivum eingesetzt. Die Verbindungen können auf an sich bekannte Weise nach den in der vorgenannten Patentanmeldung beschriebenen Verfahren oder analog zu diesen Verfahren hergestellt werden.

Es wurde nun überraschenderweise gefunden, daß Moxonidin und seine physiologisch verträglichen Säureadditionssalze nicht nur, wie in der vorstehend zitierten deutschen Patentanmeldung angegeben, für die Behandlung von Erkrankungen im Herz-Kreislauf-Bereich, wie insbesondere z.B. zur Behandlung von Bluthochdruck, geeignet sind, sondern auch zur Behandlung und/oder Prophylaxe von funktionellen Störungen und Erkrankungen der unteren Darmwege eingesetzt werden können. Moxonidin und seine physiologisch verträglichen Säureadditionssalze eignen sich hierbei insbesondere zur Behandlung und/oder Prophylaxe von mit diesen funktionellen Störungen und Erkrankungen einhergehenden abdominalen visceralen Schmerzen.

Zu den durch die erfindungsgemäß verwendeten Verbindungen behandelbaren funktionellen Störungen zählen somit insbesondere die als sogenanntes "irritable bowel syndrome" (= IBS) oder Reizdarmsyndrom bekannten Störungen der unteren Darmwege. Wesentliche Symptome von IBS sind Unterbauchschmerzen, die erheblich durch eine Übersensibilität des visceralen afferenten Nervensystems mitverursacht werden. Die erhöhte viscerale Schmerzempfindlichkeit gegenüber mechanischen oder chemischen Reizen im intestinalen Trakt führt dazu, daß IBS-Patienten schon bei physiologischen verdauungsbedingten geringen Dehnungen des Colons, z.B. bereits bei geringer Gasbildung und leichten Blähungen, die von Gesunden kaum wahrgenommen werden, starke viscerale Schmerzen erleiden. Zu den beim sogenannten "irritable bowel syndrom" (= IBS) beobachteten Symptomen gehören somit neben Motilitätsstörungen im unteren Darmbereich und Störungen des Stuhlgangs, wie insbesondere anomal beschleunigte Passage des Stuhls im Colon, daher in besonderem Maße auch viscerale Schmerzen. Die erfindungsgemäß verwendeten Verbindungen weisen hierbei eine günstige Wirkung mit ausgeprägten Wirkkomponenten gegenüber visceralen Schmerzen im Colon auf.

Störungen im gastrointestinalen Trakt können auch durch inflammatorisch bedingte Erkrankungen verursacht werden, z.B. Erkrankungen wie diese unter dem Begriff IBD (= inflammatory bowel disease) zusammengefaßt werden und die ebenfalls mit einer erhöhten visceralen Schmerzempfindlichkeit verbunden sein können.

Es wurde nun gefunden, daß Moxonidin und seine pharmakologisch akzeptablen Säureaddtitionssalze die viscerale Schmerzempfindlichkeit herabsetzen. Wegen ihrer günstigen Wirkung auf die abdominale viscerale Schmerzempfindlichkeit eignen sich Moxonidin und dessen pharmakologisch akzeptablen Säureaddtitionssalze daher insbesondere zur Behandlung und/oder Prophylaxe von abdominalen visceralen Schmerzen, wie diese insbesondere im Zusammenhang mit IBS oder IBD auftreten können.

Zur erfindungsgemäßen Behandlung von Erkrankungen und funktionellen Störungen und Erkrankungen der unteren Darmwege, insbesondere z.B. zur Behandlung und/oder Prophylaxe von damit einhergehenden visceralen Schmerzen, können Moxonidin und seine physiologisch verträglichen Säureadditionssalze in üblichen pharmazeutischen Zubereitungen oral, intravenös oder auch transdermal verabreicht werden.

So können Moxonidin und seine physiologisch verträglichen Säureadditionssalze in einer für die Behandlung von funktionellen Störungen und Erkrankungen der unteren Darmwege, insbesondere z.B. in einer zur Behandlung und/oder Prophylaxe von damit einhergehenden visceralen Schmerzen, wirksamen Menge zusammen mit üblichen pharmazeutischen Hilfs- und/oder Trägerstoffen in festen oder flüssigen pharmazeutischen Zubereitungen enthalten sein. Als Beispiele fester Präparate, die zur direkten oder verzögerten Wirkstoff-Freisetzung formuliert sein können, seien oral applizierbare Präparate wie Tabletten, Dragees, Kapseln, Pulver oder Granulate genannt, oder auch Suppositorien und Pflaster (Transdermale Therapeutische Systeme). Diese festen Präparate können pharmazeutisch übliche anorganische und/oder organische Trägerstoffe wie z.B. Milchzucker, Talkum oder Stärke neben pharmazeutisch üblichen Hilfsmitteln, beispielsweise Gleitmitteln oder Tablettensprengmitteln, enthalten. Im Falle von Pflastern wird der Wirkstoff in einem Wirkstoffreservoir, insbesondere z.B. einer Wirkstoffmatrix (z.B. eine Polymermatrix) untergebracht. Flüssige Präparate wie Lösungen, Suspensionen oder Emulsionen der Wirkstoffe können die üblichen Verdünnungsmittel wie Wasser, Öle und/oder Suspensionsmittel wie Polyethylenglykole und dergleichen enthalten. Es können zusätzlich weitere Hilfsstoffe zugegeben werden, wie z.B. Konservierungsmittel, Geschmackskorrigenzien und dergleichen.

Die Wirkstoffe können mit den pharmazeutischen Hilfs- und/oder Trägerstoffen in an sich bekannter Weise gemischt und formuliert werden. Zur Herstellung fester Arzneiformen können die Wirkstoffe beispielsweise mit den Hilfs- und/oder Trägerstoffen in üblicher Weise gemischt und naß oder trocken granuliert werden. Das Granulat oder Pulver kann direkt in Kapseln abgefüllt oder in üblicher Weise zu Tablettenkernen verpreßt werden. Diese können gewünschtenfalls in bekannter Weise dragiert werden. Pflaster bzw. Transdermale Therapeutische Systeme können in der üblichen Weise z.B. aus Abdeckfolie, Wirkstoffreservoir (selbsthaftend oder mit zusätzlicher Adhäsivschicht) und Abziehfolie sowohl als matrixgesteuerte als auch membrangesteuerte (d.h. mit zusätzlicher Kontrollmembran ausgerüstete) Systeme aufgebaut werden.

Die pharmakologischen Wirkungen von Moxonidin und dessen pharmakologisch akzeptablen Säureaddtitionssalzen im Bereich der unteren Darmwege lassen sich in pharmakologischen Standardtests an Tieren nachweisen.

<u>Beschreibung der pharmakologischen Versuchsmethoden</u>

Untersuchung der Wirkung der Verbindungen auf die viscerale Schmerzempfindlichkeit an der Ratte.

Visceraler Schmerz führt zu visceralen Reaktionen, die sich u.a. durch Kontraktionen der Bauchmuskulatur manifestieren. Die Anzahl der nach einem durch Dehnung des Dickdarms erzeugten mechanischen Schmerzreiz auftretenden Kontraktionen der Bauchmuskulatur ist somit ein Maß für die Bestimmung der visceralen Schmerzempfindlichkeit.

Die hemmende Wirkung der Testsubstanzen auf dehnungsinduzierte Bauchkontraktionen wird an Ratten geprüft. Die Dehnung des Dickdarms mit einem eingeführten Ballon wird als Reiz verwendet; als Antwort wird die Kontraktion der Bauchmuskulatur gemessen. Eine Stunde nach Sensibilisierung des Dickdarms durch Instillation von verdünnter Essigsäure (0.6 %, 1,5 ml) wird ein Latexballon eingeführt und für 10 min mit 100 mbar aufgeblasen. Während dieser Zeit werden die Kontraktionen der Bauchmuskulatur gezählt. 20 min nach subcutaner Applikation der Testsubstanz wird diese Messung wiederholt. Die Wirkung der Testsubstanz wird als prozentuale Abnahme der gezählten Kontraktionen im Vergleich zur Kontrolle errechnet. Die mit verschiedenen Dosierungen der Testsubstanz Moxonidin erzielten Verringerungen der Anzahl der Bauchkontraktionen werden in der nachfolgenden Tabelle 1 in %-Werten, bezogen auf die vor der Substanzeinnahme gemessenen Kontrollwerte (= 100 %) angegeben.

Tabelle 1

| Beeinflussung der visceralen Empfindlichkeit | |
|---|---|
| Dosis der Testsubstanz Moxonidin | Reduktion der Anzahl der dehnungsinduzierten Kontraktionen der Bauchmuskulatur in % bezogen auf Kontrollwert = 100% |
| 0,3 mg/kg | ca. 9 % (bereits leichte Wirkung) |
| 0,6 mg/kg | 23 % |
| 1,0 mg/kg | 57 % |
| 1,5 mg/kg | 71 % |

Die durch die Testsubstanz Moxonidin erzielte Abnahme der Anzahl der durch Dehnungsreiz induzierten Bauchkontraktionen ist ein deutlicher Indikator für die Wirksamkeit der Testsubstanzen gegenüber visceraler Schmerzempfindlichkeit.

Die vorstehenden Testergebnisse zeigen daher, daß Moxonidin und dessen pharmakologisch akzeptablen Säureadditionssalze die viscerale Schmerzempfindlichkeit vermindern können und deshalb für die Behandlung und/oder Prophylaxe von visceralen Schmerzen im Bereich der unteren Darmwege geeignet sind. Die genannten Verbindungen können daher vorteilhaft zur Behandlung und/Prophylaxe von funtionellen Störungen und Erkrankungen der unteren Darmwege eingesetzt werden, die im allgemeinen mit visceralen Schmerzen einhergehen, wie insbesondere z.B. IBS oder IBD.

Die zu verwendenden Dosen können individuell verschieden sein und variieren naturgemäß je nach Art des zu behandelnden Zustandes und der Applikationsform. Im allgemeinen liegen die Tagesdosen für die Behandlung von funktionellen Störungen und/oder Erkrankungen der unteren Darmwege, insbesondere z.B. zur Behandlung und/oder Prophylaxe von damit einhergehenden visceralen Schmerzen beim Menschen bei oraler Applikation im Bereich von 0,05 bis 5 mg, vorzugsweise etwa 0,25 bis 3 mg. Moxonidin oder seine Säureadditionssalze können hierbei in pharmazeutischen Zubereitungen sowohl zur sofortigen als auch verzögerten, kontrollierten und/oder gesteuerten Wirkstoff-Freisetzung verabreicht werden. Hierbei versteht es sich für den Fachmann von selbst, daß Zubereitungen mit verzögerter, kontrollierter und/oder gesteuerter Wirkstoff-Freisetzung höhere Gehalte an Wirkstoff aufweisen können als Zubereitungen zur sofortigen Wirkstoff-Freisetzung.

Das folgende Beispiel soll die Herstellung einer Moxonidin oder dessen pharmazeutisch akzeptablen Salze enthaltenden pharmazeutischen Zubereitung näher erläutern, ohne jedoch den Umfang der Anmeldung zu begrenzen.

**Beispiel 1:**

Moxonidin-haltige filmüberzogene Tabletten

**Zusammensetzung:**

| Tablettenkerne: | |
|---|---|
| Moxonidin | 0,025 Teile |
| Lactose | 9,575 Teile |
| Povidone USP | 0,070 Teile |
| Crospovidone USP | 0,300 Teile |
| Magnesiumstearat | 0,030 Teile |
| (Wasser | 0,750 Teile) |
| Gesamtfeststoff | 10,000 Teile |

| Filmüberzug: | |
|---|---|
| Hydroxypropylmethylcellulose | 0,156 Teile |
| 30%ige wäßrige Ethylcellulose-Dispersion | 0,480 Teile |
| (= Feststoff) | (0,144) Teile |
| Polyethylenglycol 6000 | 0,030 Teile |
| Titandioxid | 0,150 Teile |
| Talc | 0,1197 Teile |
| Rotes Eisenoxid | 0,0003 Teile |
| (Wasser | 3,864 Teile) |
| Gesamtfeststoff | 0,600 Teile |
| Gesamtmenge an Filmüberzugssuspension | 4,800 Teile |

Zum Überziehen von 10.000 Tablettenkerne zu je 100 mg Gewicht werden 4,8 kg der vorstehenden Filmüberzugssuspension eingesetzt.

**Tablettenkernherstellung:**

Das Moxonidin und die Lactose wurden gemischt. Die Mischung wurde mit einer Lösung des Bindemittels Povidone in Wasser durchfeuchtet, gut durchgeknetet und das erhaltene Produkt wurde auf Horden ausgebreitet und bei einer Temperatur von ca. 50 °C bis zu einem Feuchtigkeitsgehalt von höchstens 0,5 % getrocknet. Das getrocknete Produkt wurde durch ein 0,75 mm-Sieb (Frewitt-Maschine) passiert. Nach dem Vermischen des erhaltenen Granulates mit Crospovidone und Magnesiumstearat wurden daraus Tablettenkerne mit einem Gewicht von 100 mg gepreßt, so daß jeder Tablettenkern 0,25 mg Wirkstoff enthielt.

**Herstellung der Filmüberzugssuspension:**

Die Hydroxypropylmethylcellulose und das Polyethylenglycol 6.000 wurden in einem Teil des Wassers gelöst. Zu dieser Lösung wurde eine Suspension von Talc, Titandioxid und Eisenoxid in dem übrigen Wasser unter Rühren zugefügt. Die erhaltene Suspension wurde unter leichtem Rühren mit der 30%igen wäßrigen Ethylcellulose-Dispersion verdünnt.

**Filmüberziehen der Tablettenkerne:**

Die Filmüberzugssuspension wurde auf die Tablettenkerne in einer Befilmungsapparatur gesprüht, während warme Luft von ca. 70 °C die Tablettenkerne auf eine Temperatur von ca. 45 °C erwärmte. Anschließend wurden die filmüberzogenen Tabletten 16 Stunden bei einer Temperatur von ca. 45 °C getrocknet.

**Patentansprüche**

1. Verwendung von 4-Chlor-5-[(4,5-dihydro-1H-imidazol-2-yl)-amino]-6-methoxy-2-methylpyrimidin der Formel I

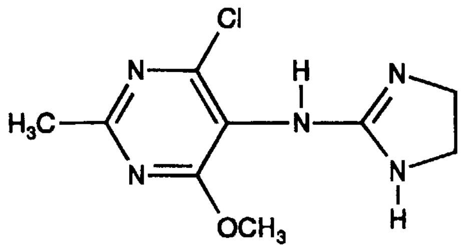

I

und dessen physiologisch verträglichen Säureadditionssalzen zur Herstellung von pharmazeutischen Zubereitungen zur Behandlung und/oder Prophylaxe von mit erhöhter Schmerzempfindlichkeit im Colonbereich verbundenen funktionellen Störungen und/oder Erkrankungen der unteren Darmwege, vorzugsweise zur Behandlung und/oder Prophylaxe von damit einhergehenden visceralen Schmerzen, in größeren Säugetieren und Menschen.

**2.** Verwendung nach Anspruch 1, dadurch gekennzeichnet, daß man die Verbindung der Formel I gemäß Anspruch 1 oder deren physiologisch verträgliche Säureadditionssalze zur Herstellung von pharmazeutischen Zubereitungen zur Behandlung und/oder Prophylaxe von abdominalen visceralen Schmerzen, die durch IBS oder IBD verursacht sind, verwendet.

**3.** Verfahren zur Herstellung von pharmazeutischen Zubereitungen zur Behandlung und/oder Prophylaxe von mit erhöhter Schmerzempfindlichkeit im Colonbereich verbundenen funktionellen Störungen und/oder Erkrankungen der unteren Darmwege, vorzugsweise zur Behandlung und/oder Prophylaxe von damit einhergehenden visceralen Schmerzen, in größeren Säugetieren und Menschen, dadurch gekennzeichnet, daß man eine zur Behandlung und/oder Prophylaxe der funktionellen Störungen und/oder Erkrankungen der unteren Darmwege, vorzugsweise eine zur Behandlung und/oder Prophylaxe von damit einhergehenden visceralen Schmerzen, wirksame Menge der Verbindung der Formel I gemäß Anspruch 1 oder deren physiologisch verträglichen Säureadditionssalzen zusammen mit üblichen pharmazeutischen Hilfsstoffen in eine geeignete Arzneiform überführt.

Europäisches Patentamt

# EUROPÄISCHER RECHERCHENBERICHT

Nummer der Anmeldung
EP 00 10 4990

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X<br><br><br><br><br>Y | DE 39 04 795 A (BEIERSDORF AG)<br>23. August 1990 (1990-08-23)<br>* Seite 24, Spalte 2 - Seite 35, Spalte 3 *<br>* Spalte 4; Beispiel 2 *<br>--- | 3<br><br><br><br><br>1,2 | A61K31/506<br>A61K31/505<br>A61P1/00 |
| Y | LIU L ET AL: "Involvement of alpha-2 adrenoceptors in the effects of moxonidine on intestinal motility and fluid transport."<br>JOURNAL OF PHARMACOLOGY AND EXPERIMENTAL THERAPEUTICS, (1997 DEC) 283 (3) 1367-74. ,<br>XP000916669<br>* Zusammenfassung *<br>* Seite 1370, linke Spalte *<br>* Seite 1371, rechte Spalte - Seite 1373 *<br>--- | 1,2 | |
| Y | SUTHERLAND L R: "Medical treatment of inflammatory bowel disease: new therapies, new drugs."<br>CMAJ, (1987 NOV 1) 137 (9) 799-802. REF: 54 ,<br>XP000916675<br>* Zusammenfassung *<br>* Seite 799, rechte Spalte *<br>* Seite 801, rechte Spalte *<br>---<br>-/-- | 1,2 | RECHERCHIERTE SACHGEBIETE (Int.Cl.7)<br>A61K<br>A61P |

Der vorliegende Recherchenbericht wurde für alle Patentansprüche erstellt

| Recherchenort | Abschlußdatum der Recherche | Prüfer |
|---|---|---|
| MÜNCHEN | 2. August 2000 | Brunnauer, H |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 00 10 4990

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| Y | SCHOLZ J ET AL: "'Treatment with clonidine in a case of the short bowel syndrome with therapy-refractory diarrhea!. Zur Behandlung mit Clonidin bei kurzdarmsyndrombedingter therapierefraktarer Diarrho." ANASTHESIOLOGIE, INTENSIVMEDIZIN, NOTFALLMEDIZIN, SCHMERZTHERAPIE, (1991 AUG) 26 (5) 265-9. , XP000916678 * Seite 265 * --- | 1,2 | |
| Y | MIDDLETON J W ET AL: "Intrathecal clonidine and baclofen in the management of spasticity and neuropathic pain following spinal cord injury: a case study." ARCHIVES OF PHYSICAL MEDICINE AND REHABILITATION, (1996 AUG) 77 (8) 824-6. , XP000916697 * Seite 824 * ----- | 1,2 | RECHERCHIERTE SACHGEBIETE (Int.Cl.7) |

Der vorliegende Recherchenbericht wurde für alle Patentansprüche erstellt

| Recherchenort | Abschlußdatum der Recherche | Prüfer |
|---|---|---|
| MÜNCHEN | 2. August 2000 | Brunnauer, H |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 00 10 4990

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

02-08-2000

| Im Recherchenbericht angeführtes Patentdokument | Datum der Veröffentlichung | Mitglied(er) der Patentfamilie | Datum der Veröffentlichung |
|---|---|---|---|
| DE 3904795 A | 23-08-1990 | KEINE | |

EPO FORM P0461

Für nähere Einzelheiten zu diesem Anhang : siehe Amtsblatt des Europäischen Patentamts, Nr.12/82